# EUROPEAN PATENT SPECIFICATION

(11) **EP 4 215 144 B1**
(45) Date of publication and mention of the grant of the patent: **20.05.2026**
(21) Application number: 23152482.8
(22) Date of filing: 19.01.2023
(51) Int. Cl.: A61B 18/14, A61B 5/287

(54) **ELECTRODE ASSEMBLIES OF A BASKET CATHETER HAVING MECHANICAL RETAINERS AND METHODS OF THE SAME**
ELEKTRODENANORDNUNGEN EINES KORBKATHETERS MIT MECHANISCHEN HALTERN UND VERFAHREN DAFÜR
ENSEMBLES D'ÉLECTRODES D'UN CATHÉTER À PANIER AYANT DES ÉLÉMENTS DE RETENUE MÉCANIQUES ET PROCÉDÉS ASSOCIÉS

(30) Priority: 20.01.2022 US 202263301146 P; 14.12.2022 US 202218066117
(43) Date of publication of application: 26.07.2023
(73) Proprietor: Biosense Webster (Israel) Ltd., Yokneam 2066717 (IL)
(72) Inventor: OKARSKI, Kevin Mark, Irvine, 92618 (US); DATTA, Keshava, Irvine, 92618 (US); BAH, Abubakarr, Irvine, 92618 (US); NGUYEN, Thanh, Irvine, 92618 (US)
(74) Representative: Carpmaels & Ransford LLP

(56) References cited:
- WO-A1-2021/126980
- US-A- 5 499 981
- US-A1- 2015 366 508
- US-A1- 2021 093 376

## Description

### FIELD

The present invention relates generally to medical devices, and in particular catheters with electrodes, and further relates to, but not exclusively, catheters suitable for use to induce irreversible electroporation (IRE) of cardiac tissues.

### BACKGROUND

Cardiac arrhythmias, such as atrial fibrillation (AF), occur when regions of cardiac tissue abnormally conduct electric signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art tend to utilize radiofrequency (RF) electrical energy to heat tissue. RF ablation can have certain drawbacks, such as heightened risk of thermal cell injury which can lead to tissue charring, burning, steam pop, phrenic nerve palsy, pulmonary vein stenosis, and esophageal fistula. Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent Pub. No. 2021/0169550A1, 2021/0169567A1, 2021/0169568A1, 2021/0161592A1, 2021/0196372A1, 2021/0177503A1, and 2021/0186604A1.

Regions of cardiac tissue can be mapped by a catheter to identify the abnormal electrical signals. The same or different catheter can be used to perform ablation. Some example catheters include a number of spines with electrodes positioned thereon. The electrodes are generally attached to the spines and secured in place by soldering, welding, or using an adhesive. Due to the small size of the spines and the electrodes, however, soldering, welding, or adhering the electrodes to the spines can be a difficult task, increasing the manufacturing time and cost and the chances that the electrode fails due to an improper bond or misalignment. What is needed, therefore, are systems and methods of attaching an electrode to a spine of a basket assembly without the need for soldering, welding, or using adhesive.

WO 2021/126980 A1 describes a pulmonary vein isolation catheter that includes a tip section having an expandable portion and a deployment member. The expandable portion includes a plurality of mesh electrode panels that are electrically insulated from one another. The expandable portion is mechanically coupled to (i) the deployment member at a distalmost portion of the tip section and (ii) a distal end portion of a catheter shaft. The expandable portion is expandable and compressible via proximal and distal movement, respectively, of the deployment member. In some embodiments, the expandable portion in a deployed state is pear- or onion-shaped and includes a nose portion and/or an active body portion. The nose portion can be insulated and or configured to fit within a pulmonary vein and position the active body portion against tissue about the ostium of the pulmonary vein. In an embodiment, mesh electrode panels are attached to form the expandable portion of the tip section. Eyelets of adjacent mesh electrode panels are aligned (overlapped) and held together with a fastener. In an embodiment, the fastener is a rivet having a primary head. In such implementations, the eyelets of adjacent mesh electrode panels can be aligned with one another such that a primary head of a fastener passes through the aligned eyelets. In other embodiments, a different type of fastener (e.g., a two-headed rivet, a crimp, a flange screw and washer, a PEM^{®} fastener, etc.) can be used. The primary head of at least some of the fasteners can accommodate and/or include a sensor. In an embodiment, each sensor can act as an electrode (e.g., a surface electrode) to detect electrical activity of the heart in an area local to the sensor.

### SUMMARY

The present invention provides medical probes and methods of constructing such probes, in accordance with the appended claims.

### BRIEF DESCRIPTION OF THE DRAWINGS

FIG. 1 is a schematic pictorial illustration of a medical system comprising a medical probe whose distal end comprises a basket assembly with electrodes, in accordance with an embodiment of the present invention;
FIG. 2A is a schematic pictorial illustration showing a perspective view of a medical probe in an expanded form, in accordance with an embodiment of the present invention;
FIG. 2B is a schematic pictorial illustration showing a side view of a medical probe in a collapsed form, in accordance with the disclosed technology;
FIGs. 3A and 3B are schematic pictorial illustrations showing exploded views of a tubular shaft and spines of the basket assembly to illustrate how the spines can be assembled together with the tubular shaft, in accordance with an embodiment of the present invention;
FIGs. 4A and 4B are schematic pictorial illustrations showing perspective views of an electrode attached to a spine, in accordance with an embodiment of the present invention;
FIG. 5 is a schematic pictorial illustration showing a side exploded view of an electrode assembly and a spine, in accordance with an embodiment of the present invention;
FIG. 6 is a schematic pictorial illustration showing a perspective exploded view of an electrode assembly and a spine, in accordance with an embodiment of the present invention;
FIG. 7A is a schematic pictorial illustration showing a top view of a spine, in accordance with another embodiment of the present invention;
FIG. 7B is a schematic pictorial illustration showing a side view of an electrode, in accordance with another embodiment of the present invention;
FIG. 7C is a schematic pictorial illustration showing a perspective view of a washer, in accordance with another embodiment of the present invention;
FIG. 7D is a schematic pictorial illustration showing a perspective view of another washer, in accordance with another embodiment of the present invention;
FIGs. 8A and 8B are schematic pictorial illustrations showing perspective views of an electrode assembly, in accordance with another embodiment of the present invention;
FIG. 9 is a schematic pictorial illustration showing an exploded view of an electrode assembly, in accordance with another embodiment of the present invention;
FIGs. 10A and 10B are schematic pictorial illustrations showing perspective views of a housing portion of an electrode assembly, in accordance with another embodiment of the present invention;
FIGs. 10C and 10D are schematic pictorial illustrations showing perspective views of another housing portion of an electrode assembly, in accordance with another embodiment of the present invention;
FIGs. 11A and 11B are schematic pictorial illustrations showing various insulative jackets of a given medical device, in accordance with embodiments of the present invention;
FIGs. 12A and 12B are schematic pictorial illustrations showing a side view of a spine of a given medical device, in accordance with embodiments of the present invention;
FIGs. 13A and 13B are schematic pictorial illustrations showing cross-sectional views of a given wire of a medical probe, in accordance with an embodiment of the present invention;
FIG. 14 is a flowchart illustrating a method of assembling a basket assembly, in accordance with an embodiment of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "biphasic pulse" and "monophasic pulse" refer to respective electrical signals. "Biphasic pulse" refers to an electrical signal having a positive-voltage phase pulse (referred to herein as "positive phase") and a negative-voltage phase pulse (referred to herein as "negative phase"). "Monophasic pulse" refers to an electrical signal having only a positive or only a negative phase. Preferably, a system providing the biphasic pulse is configured to prevent application of a direct current voltage (DC) to a patient. For instance, the average voltage of the biphasic pulse can be zero volts with respect to ground or other common reference voltage. Additionally, or alternatively, the system can include a capacitor or other protective component. Where voltage amplitude of the biphasic and/or monophasic pulse is described herein, it is understood that the expressed voltage amplitude is an absolute value of the approximate peak amplitude of each of the positive-voltage phase and/or the negative-voltage phase. Each phase of the biphasic and monophasic pulse preferably has a square shape having an essentially constant voltage amplitude during a majority of the phase duration. Phases of the biphasic pulse are separated in time by an interphase delay. The interphase delay duration is preferably less than or approximately equal to the duration of a phase of the biphasic pulse. The interphase delay duration is more preferably about 25% of the duration of the phase of the biphasic pulse.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The term "temperature rating", as used herein, is defined as the maximum continuous temperature that a component can withstand during its lifetime without causing thermal damage, such as melting or thermal degradation (e.g., charring and crumbling) of the component.

The present disclosure is related to systems, method or uses and devices which utilize end effectors having electrodes affixed to spines. Example systems, methods, and devices of the present disclosure may be particularly suited for IRE ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation.

RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore cryoablation is not viable in certain anatomical geometries which may be reached by electrical ablation devices.

IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

Electroporation can be induced by applying a pulsed electric field across biological cells to cause reversable (temporary) or irreversible (permanent) creation of pores in the cell membrane. The cells have a transmembrane electrostatic potential that is increased above a resting potential upon application of the pulsed electric field. While the transmembrane electrostatic potential remains below a threshold potential, the electroporation is reversable, meaning the pores can close when the applied pulse electric field is removed, and the cells can self-repair and survive. If the transmembrane electrostatic potential increases beyond the threshold potential, the electroporation is irreversible, and the cells become permanently permeable. As a result, the cells die due to a loss of homeostasis and typically die by apoptosis. Generally, cells of differing types have differing threshold potential. For instance, heart cells have a threshold potential of approximately 500 V/cm, whereas for bone it is 3000 V/cm. These differences in threshold potential allow IRE to selectively target tissue based on threshold potential.

The solution of this disclosure includes systems and methods for applying electrical signals from catheter electrodes positioned in the vicinity of myocardial tissue, preferably by applying a pulsed electric field effective to induce electroporation in the myocardial tissue. The systems and methods can be effective to ablate targeted tissue by inducing irreversible electroporation. In some examples, the systems and methods can be effective to induce reversible electroporation as part of a diagnostic procedure. Reversible electroporation occurs when the electricity applied with the electrodes is below the electric field threshold of the target tissue allowing cells to repair. Reversible electroporation does not kill the cells but allows a physician to see the effect of reversible electroporation on electrical activation signals in the vicinity of the target location. Example systems and methods for reversible electroporation is disclosed in U.S. Patent Publication 2021/0162210.

The pulsed electric field, and its effectiveness to induce reversible and/or irreversible electroporation, can be affected by physical parameters of the system and biphasic pulse parameters of the electrical signal. Physical parameters can include electrode contact area, electrode spacing, electrode geometry, etc. examples presented herein generally include physical parameters adapted to effectively induce reversible and/or irreversible electroporation. Biphasic pulse parameters of the electrical signal can include voltage amplitude, pulse duration, pulse interphase delay, inter-pulse delay, total application time, delivered energy, etc. In some examples, parameters of the electrical signal can be adjusted to induce both reversible and irreversible electroporation given the same physical parameters. Examples of various systems and methods of ablation including IRE are presented in U.S. Patent Publications 2021/0169550A1, 2021/0169567A1, 2021/0169568A1, 2021/0161592A1, 2021/0196372A1, 2021/0177503A1, and 2021/0186604A1.

To deliver pulsed field ablation (PFA) in an IRE (irreversible electroporation) procedure, electrodes should contact the tissue being ablated with a sufficiently large surface area. As described hereinbelow, the medical probe includes a flexible insertion tube having proximal and distal ends, and a basket assembly at the distal end of the flexible insertion tube. The basket assembly includes at least one spine and a plurality of electrode assemblies. Each electrode assembly can be configured to interlock with the spine to prevent the spine from sliding proximally or distally along the length of the spine.

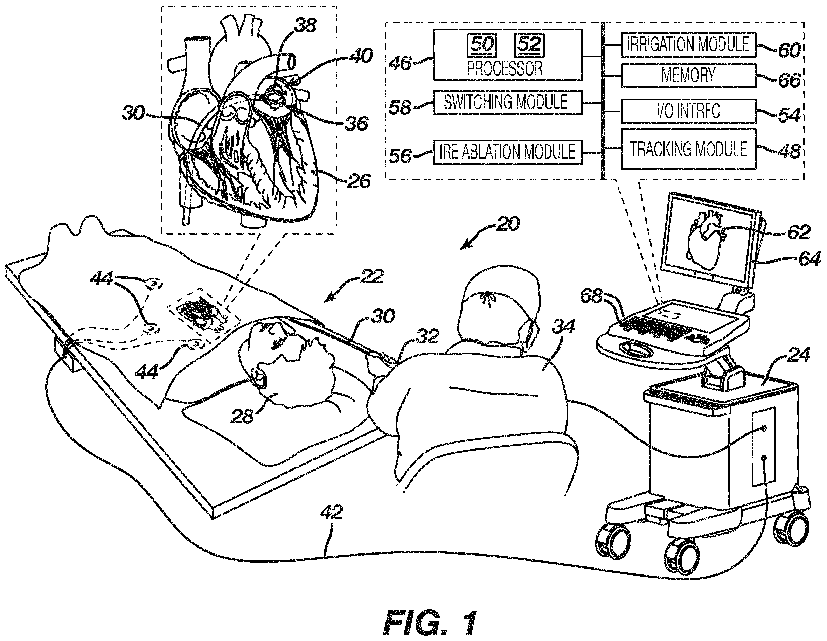

FIG. 1 is a schematic, pictorial illustration of a medical system 20 including a medical probe 22 and a control console 24, in accordance with an embodiment of the present invention. Medical system 20 may be based, for example, on the CARTO^{®} system, produced by Biosense Webster Inc. of 31 Technology Drive, Suite 200, Irvine, CA 92618 USA. In embodiments described hereinbelow, medical probe 22 can be used for diagnostic or therapeutic treatment, such as for performing ablation procedures in a heart 26 of a patient 28. Alternatively, medical probe 22 may be used, mutatis mutandis, for other therapeutic and/or diagnostic purposes in the heart or in other body organs.

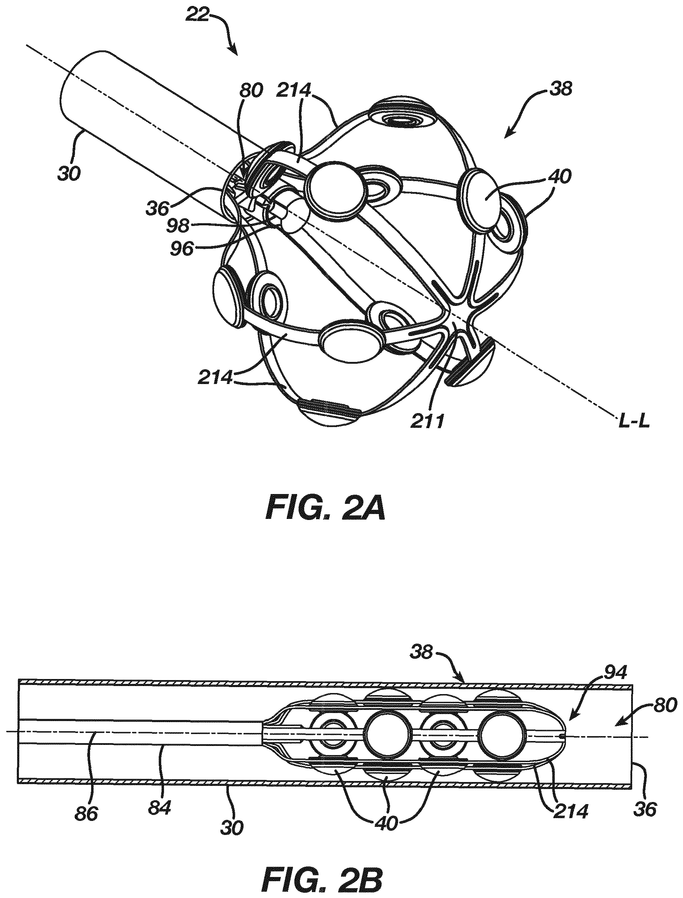

Medical probe 22 includes a flexible insertion tube 30 and a handle 32 coupled to a proximal end of the insertion tube. During a medical procedure, a medical professional 34 can insert probe 22 through the vascular system of patient 28 so that a distal end 36 of the medical probe enters a body cavity such as a chamber of heart 26. Upon distal end 36 entering the chamber of heart 26, medical professional 34 can deploy a basket assembly 38 affixed to distal end 36. Basket assembly 38 can include a plurality of electrodes 40 affixed to a plurality of spines, as described in the description referencing FIGs. 2A and 2B hereinbelow. To start performing a medical procedure such as irreversible electroporation (IRE) ablation, medical professional 34 can manipulate handle 32 to position distal end 36 so that electrodes 40 engage cardiac tissue at a desired location or locations. Upon positioning the distal end 36 so that electrodes 40 engages cardiac tissue, the medical professional 34 can activate the medical probe 22 such that electrical pulses are delivered by the electrodes 40 to perform the IRE ablation.

In the configuration shown in FIG. 1, control console 24 is connected, by a cable 42, to body surface electrodes, which typically include adhesive skin patches 44 that are affixed to patient 28. Control console 24 includes a processor 46 that, in conjunction with a tracking module 48, determines location coordinates of distal end 36 inside heart 26. Location coordinates can be determined based on electromagnetic position sensor output signals provided from the distal portion of the catheter when in the presence of a generated magnetic field. Location coordinates can additionally, or alternatively be based on impedances and/or currents measured between adhesive skin patches 44 and electrodes 40 that are affixed to basket assembly 38. In addition to being used as location sensors during a medical procedure, electrodes 40 may perform other tasks such as ablating tissue in the heart.

As described hereinabove, in conjunction with tracking module 48, processor 46 may determine location coordinates of distal end 36 inside heart 26 based on impedances and/or currents measured between adhesive skin patches 44 and electrodes 40. Such a determination is typically after a calibration process relating the impedances or currents to known locations of the distal end has been performed. While embodiments presented herein describe electrodes 40 that are preferably configured to deliver IRE ablation energy to tissue in heart 26, configuring electrodes 40 to deliver any other type of ablation energy to tissue in any body cavity is possible. Furthermore, although described in the context of being electrodes 40 that are configured to deliver IRE ablation energy to tissue in the heart 26, one skilled in the art will appreciate that the disclosed technology can be applicable to electrodes used for mapping and/or determining various characteristics of an organ or other part of the patient's 28 body.

Processor 46 may include real-time noise reduction circuitry 50 typically configured as a field programmable gate array (FPGA), followed by an analog-to-digital (A/D) signal conversion integrated circuit 52. The processor can be programmed to perform one or more algorithms and uses circuitry 50 and circuit 52 as well as features of modules to enable the medical professional 34 to perform the IRE ablation procedure.

Control console 24 also includes an input/output (I/O) communications interface 54 that enables control console 24 to transfer signals from, and/or transfer signals to electrodes 40 and adhesive skin patches 44. In the configuration shown in FIG. 1, control console 24 additionally includes an IRE ablation module 56 and a switching module 58.

IRE ablation module 56 is configured to generate IRE pulses having peak power in the range of tens of kilowatts. In some examples, the electrodes 40 are configured to deliver electrical pulses having a peak voltage of at least 900 volts (V) and in some instances 1100V or higher. The medical system 20 performs IRE ablation by delivering IRE pulses to electrodes 40. Preferably, the medical system 20 delivers biphasic pulses between electrodes 40 on the spine. Additionally, or alternatively, the medical system 20 delivers monophasic pulses between at least one of the electrodes 40 and a skin patch.

Where irrigation is desired, system 20 supplies irrigation fluid (e.g., a saline solution) to distal end 36 and to the electrodes 40 via a channel (not shown) in insertion tube 30. Control console 24 includes an irrigation module 60 to monitor and control irrigation parameters, such as the pressure and the temperature of the irrigation fluid.

Based on signals received from electrodes 40 and/or adhesive skin patches 44, processor 46 can generate an electroanatomical map 62 that shows the location of distal end 36 in the patient's body. During the procedure, processor 46 can present map 62 to medical professional 34 on a display 64, and store data representing the electroanatomical map in a memory 66. Memory 66 may include any suitable volatile and/or non-volatile memory, such as random-access memory or a hard disk drive.

In some embodiments, medical professional 34 can manipulate map 62 using one or more input devices 68. In alternative embodiments, display 64 may include a touchscreen that can be configured to accept inputs from medical professional 34, in addition to presenting map 62.

FIG. 2A is a schematic pictorial illustration showing a perspective view of a medical probe 22 with a basket assembly 38 in an expanded form when unconstrained, such as by being advanced out of an insertion tube lumen 80 at a distal end 36 of an insertion tube 3. FIG. 2B shows the basket assembly in a collapsed form within insertion tube 30. In the expanded form (FIG. 2A), the spines 214 bow radially outwardly and in the collapsed form (FIG. 2B) the spines are arranged generally along a longitudinal axis 86 of insertion tube 30.

As shown in FIG. 2A, basket assembly 38 includes a plurality of flexible spines 214 that are formed at the end of a tubular shaft 84 and are connected at both ends. During a medical procedure, medical professional 34 can deploy basket assembly 38 by extending tubular shaft 84 from insertion tube 30 causing the basket assembly 38 to exit the insertion tube and transition to the expanded form. Spines 214 may have elliptical (e.g., circular) or rectangular (that may appear to be flat) cross-sections, and include a flexible, resilient material (e.g., a shape-memory alloy such as nickel-titanium (also known as Nitinol), cobalt chromium, or any other suitable material).

In embodiments described herein, electrodes 40 can be configured to deliver ablation energy (RF and/or IRE) to tissue in heart 26. Additionally, or alternatively, the electrodes can also be used to determine the location of basket assembly 38 and/or to measure a physiological property such as local surface electrical potentials at respective locations on tissue in heart 26. The electrodes 40 can be biased such that a greater portion of the electrode 40 faces outwardly from the basket assembly 39 such that the electrodes 40 deliver a greater amount of electrical energy outwardly away from the basket assembly 38 (i.e., toward the heart 26 tissue) than inwardly toward the basket assembly 38.

Examples of materials ideally suited for forming electrodes 40 include gold, platinum, and palladium (and their respective alloys). These materials also have high thermal conductivity which allows the minimal heat generated on the tissue (i.e., by the ablation energy delivered to the tissue) to be conducted through the electrodes to the back side of the electrodes (i.e., the portions of the electrodes on the inner sides of the spines), and then to the blood pool in heart 26.

Basket assembly 38 has a distal end 94 and includes a stem 96 that extends longitudinally from a distal end 36 of tubular shaft 84 towards distal end 94 of basket assembly 38. As described supra, control console 24 includes irrigation module 60 that delivers irrigation fluid to distal end 36. Stem 96 includes multiple irrigation openings 98, wherein each given irrigation opening 98 can be angled to spray or otherwise disperse of the irrigation fluid to either a given electrode 40 or to tissue in heart 26.

Since electrodes 40 do not include irrigation openings that deliver irrigation fluid, the configuration described hereinabove enables heat to be transferred from the tissue (i.e., during an ablation procedure) to the portion of the electrodes on the inner side of the spines 214, and the electrodes 40 can be cooled by aiming the irrigation fluid, via irrigation openings 98, at the portion of the electrodes 40 on the inner side of the spines 214.

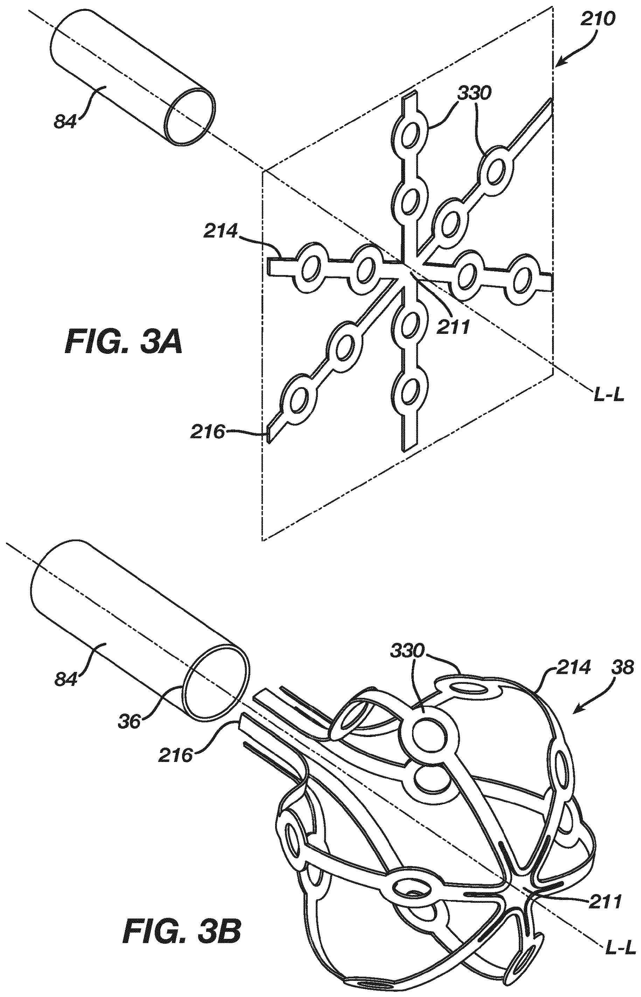

FIGs. 3A and 3B are schematic pictorial illustrations showing exploded views of a tubular shaft 84 and spines 214 of the basket assembly to provide one example of how the spines 214 can be assembled together with the tubular shaft 84, in accordance with an embodiment of the present invention. As shown in FIG. 3A, the spines 214 can be formed from a single sheet of planar material to form a generally star shape. In other words, the spines 214 can be formed from the single sheet of planar material such that the spines 214 converge toward a central intersection 211. The intersection 211 can be a solid piece of material (as shown in FIG. 3A) or include one or more apertures (as shown in FIG. 3B).

The spines 214 can be folded or otherwise bent such that a proximal end 216 of the spines 214 can be inserted into the distal end 85 of the tubular shaft 84 as shown in FIG. 3B. Although not shown in FIGs. 3A and 3B, it will be appreciated that the electrodes 40 can be attached to the spines 214 before the spines are inserted into the tubular shaft 84 to form the basket assembly 38. As stated previously, the spines 214 can include a flexible, resilient material (e.g., a shape-memory alloy such as nickel-titanium, also known as Nitinol) that can enable the basket assembly 38 to transition from its collapsed form (as shown in FIG. 2B) to its expanded form (as shown in FIG. 2A) when the basket assembly 38 is deployed from insertion tube 30. As will become apparent throughout this disclosure, the spine 214 can be electrically isolated from the electrode 40 to prevent arcing from the electrode 40 to the spine 214.

As will be appreciated by one skilled in the art with the benefit of this disclosure, the basket assembly 38 shown in FIGs. 2A-3B having spines 214 formed from a single sheet of planar material and converging at a central intersection is offered merely for illustrative purposes and the disclosed technology can be applicable to other configurations of basket assemblies 38. For example, the disclosed technology can be applicable to basket assemblies 38 formed from a single spine 214 or multiple spines 214 with each spine 214 being attached at both ends. In other examples, the basket assembly 38 can include a central hub connecting the multiple spines 214 together at a distal end 94 of the basket assembly 38. In yet other examples, the basket assembly 38 can include a single spine 214 configured to form a spiral, multiple spines 214 configured to form a spiral, multiple spines 214 configured to form a tripod or multiple tripods, or any other shape of basket assembly 38. As well, the spine assembly 210 can be formed by laser cutting a cylindrical hollow stock material whereby the laser is mounted for rotation about the longitudinal axis (and translation thereto) of the cylindrical stock while cutting through the cylindrical stock. Thus, although FIGs. 2A-3B illustrate a specific configuration of basket assembly 38, the disclosed technology should not be construed as so limited.

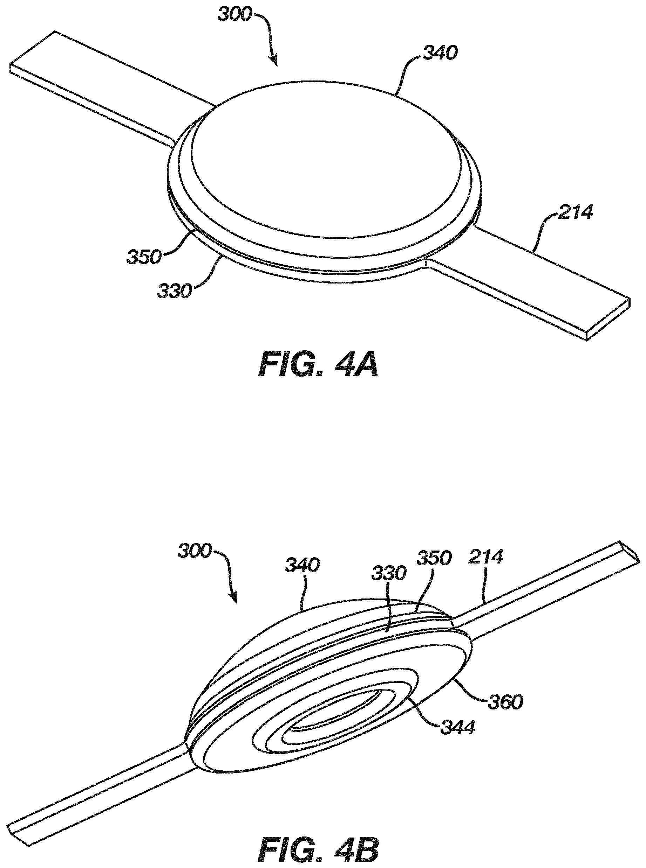

Turning now to FIGs. 4A-10D, various examples of electrode assemblies 300, 800 will be described. As will become apparent throughout this disclosure, the electrode assemblies 300, 800 can be configured to attach the electrodes 340, 840 to the spines 214 and prevent the electrodes 340, 840 from sliding proximally or distally along the length of the spine 214. Generally speaking, the electrode assemblies 300, 800 can include an electrode 340, 840, a first electrically insulating portion (i.e., a first washer 350 or a first insulated housing portion 870, as described herein), and a second electrically insulating portion (i.e., a second washer 360 or a second insulated housing portion 880 , as described herein). The first insulating portion and the second insulating portion can electrically isolate the electrode 340, 840 from the spine 214. As will be appreciated, although the electrode assemblies 300, 800 are described as having a first and a second electrically insulating portion, one of skill in the art will appreciate that insulative coatings can be used in addition to, or in place of, the insulating portions described herein.

The electrode assemblies 300, 800 are preferably asymmetrical with respect to a plane of the spine such that the electrode 340 presents an electrically conductive surface area facing outwardly from a periphery of the basket of the catheter and an insulating surface faces inwardly from the periphery of the basket. Furthermore, as will be appreciated by one of skill in the art, the electrodes 340, 840 can be the same as, or in place of, the electrode 40 previously described herein. That is, the electrode assemblies 300, 800 are examples of the present disclosure that can be used to attach the electrodes 340, 840 to the spine 214 to facilitate the electrodes 340, 840 performing the same or a similar function as the electrodes 40 described herein (e.g., delivering IRE ablation energy to cardiac tissue). One of skill in the art will appreciate that the electrode assemblies 300, 800 described herein are offered for illustrative purposes and that other electrode assemblies having similar features can reasonably be construed as falling within the scope of this disclosure. Thus, the present disclosure should not be construed as limited to the particular electrode assemblies 300, 800 shown in FIGs. 4A-10D and described herein.

FIGs. 4A and 4B are schematic pictorial illustrations showing perspective views of an electrode assembly 300 attached to a spine 214, in accordance with an embodiment of the present invention. The electrode assembly 300 can include an electrode 340, a first washer 350, and a second washer 360. The electrode 340 can include electrically conductive material as described in relation to the electrode 40 (e.g., gold, platinum, and palladium (and their respective alloys)). The first washer 350 and the second washer 360 can be made from a biocompatible, electrically insulative material such as polyetherimide (Ultem), polyimide, polyetheretherketon (PEEK), polytetraflouroethylene (PTFE), flourinated ethylene propylene (FEP), etc. Furthermore, the first washer 350 and the second washer 360 can include filler materials such as PTFE (for insulation), boron nitride (for insulation), diamond (for thermal conductivity), etc. In this way, the first washer 350 and the second washer 360 can be configured to electrically isolate the electrode 340 from the spine 214 to prevent or reduce arcing between the electrode 340 and the spine 214.

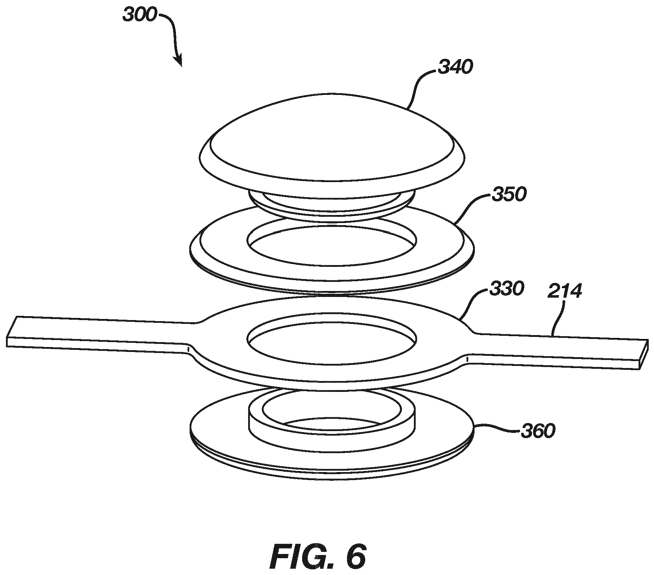

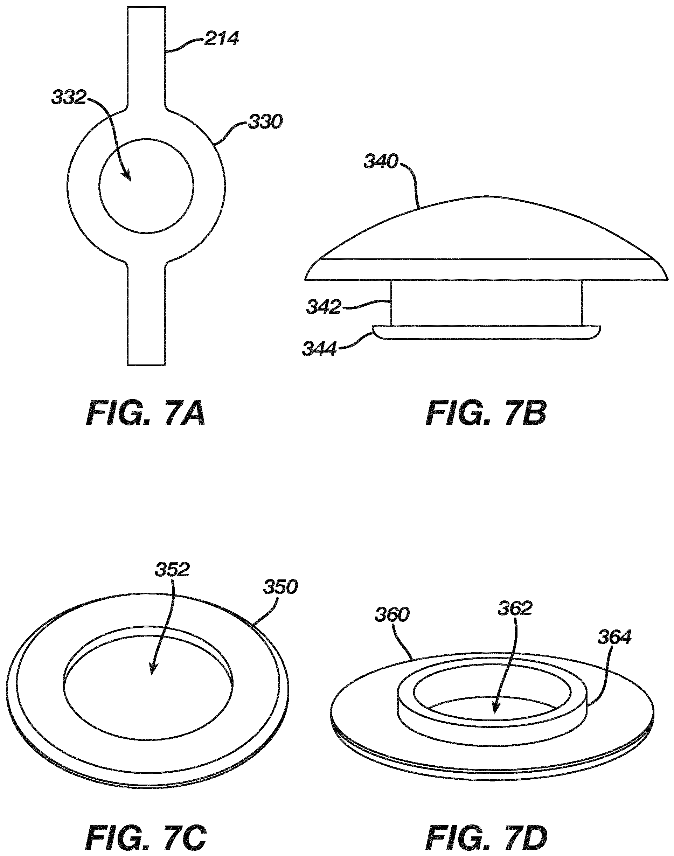

The electrode 340, the first washer 350, and the second washer 360 can be configured to interlock with each other to form the electrode assembly 300. Furthermore, as will be described in greater detail herein, the electrode 340, the first washer 350, and the second washer 360 can be configured to interlock with a spine 214 to prevent the electrode assembly 300 from sliding proximally or distally along the length of the spine 214. To help prevent the electrode assembly 300 from sliding proximally or distally along the length of the spine 214, the spine 214 can include a retainer ring 330 that can be a ring formed in the spine 214 comprising an aperture 332 through which portions of the electrode assembly 300 can pass. The retainer ring 330 may be better observed in, and is described in relation to, FIGs. 6 and 7A.

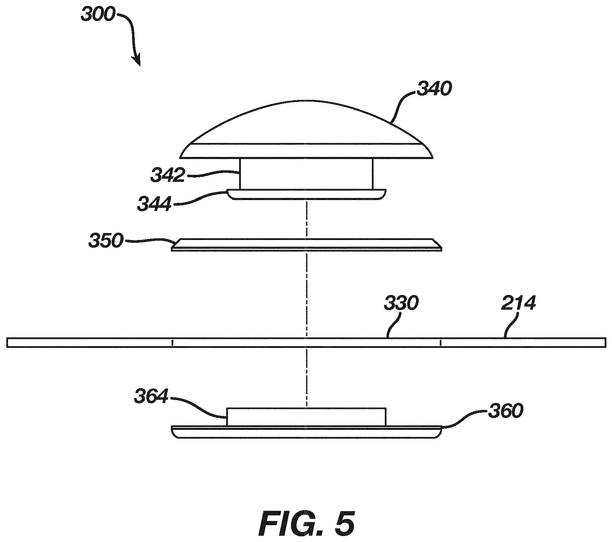

FIG. 5 is a schematic pictorial illustration showing a front exploded view of an electrode assembly 300 and a spine 214 while FIG. 6 is a schematic pictorial illustration showing a perspective exploded view of an electrode assembly 300 and a spine 214, in accordance with an embodiment of the present invention. As illustrated in FIGs. 5 and 6, the electrode 340 and the first washer 350 can be positioned on a first side of the spine 214 while the second washer 360 can be positioned on a second side of the spine 214. Thus, when the electrode 340, the first washer 350, and the second washer 360 are pushed together to be attached to the spine 214, the spine 214 can be positioned between portions of the electrode assembly 300. Furthermore, as illustrated in FIG. 6, at least a portion of the electrode 340 can be configured to pass through the aperture 332 of the retainer ring 330 to help secure the electrode assembly 300 to the spine 214. In other words, because at least a portion of the electrode 340 can pass through the retainer ring 330, the electrode assembly 300 can be secured together through the retainer ring 330 such that the electrode assembly 300 is prevented from becoming disengaged with, or sliding along, the spine 214.

The electrode assembly 300 can be secured in place by including a mechanical interference that can prevent the electrode assembly 300 from separating once secured together. For example, the electrode 340 and at least the second washer 360 can be configured to form an interference fit together when pressed together to prevent the electrode 340 from becoming disengaged with the second washer 360. For example, an outer diameter of a protrusion of 342 of the electrode 340 can be slightly larger than the inner diameter of an aperture 362 through the second washer 360 to form an interference fit between the electrode 340 and the second washer 360 when pressed together.

As another example, the electrode 340 can include a swage fitting 344 that can be sized to secure the electrode assembly 300 in place once the electrode 340 is engaged with the second washer 360. The swage fitting 344 can be formed as the electrode 340 is pushed together with the second washer 360 with use of a tool adapted to form a swage fitting. Alternatively, or in addition, the swage fitting 344 can be formed prior to pressing the electrode 340 and the second washer 360 together. For example, the swage fitting 344, the second washer 360, or both, can comprise a resilient material that can deflect as the swage fitting 344 is pushed through an aperture 362 of the second washer 360. The swage fitting 344 can then return (or nearly) to its original configuration after being pushed through the aperture 362 of the second washer 360 such that the electrode assembly 300 is secured in place. In other words, the swage fitting 344 and the aperture 362 of the second washer 360 can both be configured to allow a portion of the electrode 340 to pass through the aperture 362 of the second washer 360 in a first direction but prevent the electrode 340 from passing through the aperture 362 in a second direction and becoming disengaged with the second washer 360 after assembly. As will be appreciated by one of skill in the art, although the electrode assembly 300 is illustrated as having a second washer 360 having an aperture 362 configured to receive at least a portion of an electrode 340 to secure the electrode assembly 300 in place, the disclosed technology is not so limited. As a non-limiting example, the configuration of the electrode assembly 300 may be switched and the electrode 340 may comprise an aperture (or multiple apertures) configured to receive a protrusion of the second washer 360 to secure electrode assembly 300 in place.

FIG. 7A is a schematic pictorial illustration showing a top view of the spine 214. As illustrated in FIG. 7A, the spine 214 can include a retainer ring 330 that can be a simple ring or circle formed in the spine 214. The retainer ring 330 can include an aperture 332 through which at least a portion of the electrode assembly 300 can pass or be positioned. As an example, and as illustrated in FIG. 7B, the electrode 340 can include a protrusion 342 that can be configured to extend through the aperture 332 of the retainer ring 330 when the electrode assembly 300 is attached to the spine 214. In this way, when the electrode 340 is engaged with the second washer 360, the electrode assembly 300 can be prevented from sliding along, or otherwise becoming disengaged with, the spine 214. As shown in FIG. 7B, the protrusion 342 of the electrode 340 can extend outwardly from the electrode 340 and include the swage fitting 344 as previously described. The protrusion 342 can be sized to fit in the aperture 332 of the retainer ring 330 as well as an aperture 352 of the first washer 350 and the aperture 362 of the second washer 360. The electrode 340 can be connected to a wire that passes through the back side of the electrode 340 proximate the swage fitting 344. The wire, for example, can be welding, crimped, soldered, or otherwise connected to the electrode by passing through the protrusion 342 and connecting to the interior surface of the electrode 340.

As shown in FIG. 7D, the second washer 360 can further include a lip 364 that can be configured to extend through the aperture 332 of the retainer ring 330. The lip 364 can also be sized to extend through the aperture 352 of the first washer 350 such that the electrode 340 is electrically isolated from the spine 214.

Although the electrode assembly 300 and its components are illustrated in FIGs. 4A-7D as having a generally cylindrical shape, one of skill in the art will appreciate that electrode assemblies 300 having other shapes are contemplated. For example, the electrode assembly 300 can comprise a generally rectangular, triangular, polygonal, oval, or any other suitable shape without departing from the scope of this disclosure. Furthermore, the retainer ring 330, the protrusion 342 of the electrode 340, the aperture 352 of the first washer 350, and the aperture 362 of the second washer 360 can each comprise a shape other than a generally circular shape as shown (i.e., a generally rectangular, triangular, polygonal, oval, or any other suitable shape). Thus, although the electrode assembly 300 is shown as described as having a particular shape and configuration, one of skill in the art will appreciate that the electrode assembly 300 can comprise various other shapes and configuration depending on the particular application without departing from the scope of this disclosure.

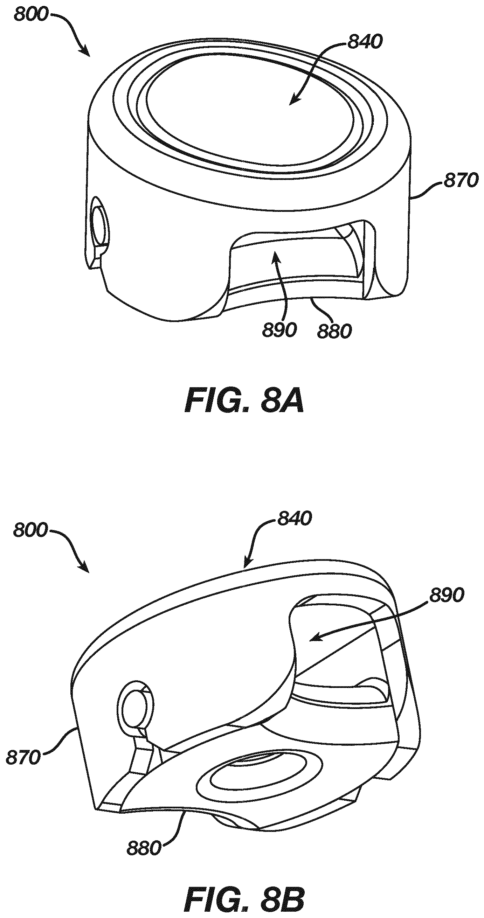

Turning now to FIGs. 8A-10D, another example electrode assembly 800 is shown and herein described. The electrode assembly 800 can be configured to perform a similar function as the electrode assembly 300 described herein. That is, the electrode assembly 800 can be configured to secure the electrode 840 to a spine 214 and the electrode 840 can be configured to deliver IRE ablation energy to cardiac tissue, among the other features herein described. The electrode assembly 800 can include an electrode 840, a first insulated housing portion 870, and a second insulated housing portion 880. The electrode 840 can include electrically conductive material as described in relation to the electrode 40 and the electrode 340 (e.g., gold, platinum, and palladium (and their respective alloys)). The first insulated housing portion 870 and the second insulated housing portion 880 can include a material that can electrically isolate the electrode 840 from the spine 214. For example, the first insulated housing portion 870 and the second insulated housing portion 880 can be made from a biocompatible, electrically insulative material such as Ultem, PEEK, liquid-crystaline polymer (Vectra/Zenite), polycarbonate, etc. The first insulated housing portion 870 and the second insulated housing portion 880 can include filler materials such as glass fiber having a concentration of approximately 2-30%. In this way, the first insulated housing portion 870 and the second insulated housing portion 880 can be configured to electrically isolate the electrode 840 from the spine 214 to prevent or reduce arcing between the electrode 840 and the spine 214.

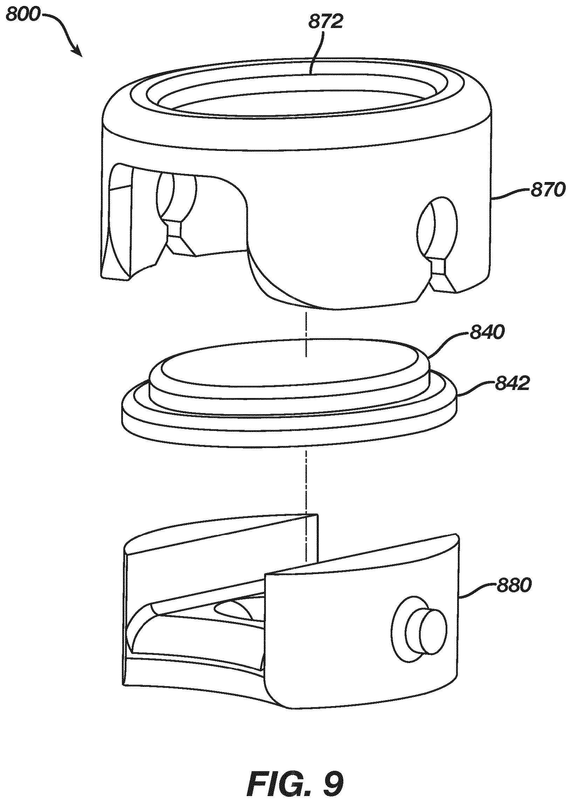

The electrode assembly 800 can include a channel 890 through which at least a portion of the spine 214 can pass. For example, the channel 890 can be sized to at least partially receive the spine 214 such that the electrode assembly 800 can be secured to the spine 214 by clamping around the spine 214. For example, although not shown in FIG. 9, the spine 214 may be placed between the electrode 840 and the second insulated housing portion 880 such that when the electrode assembly 800 is pushed together to be assembled, the electrode assembly 800 can be secured around the spine 214. To further secure the electrode assembly 800 to the spine, a cross-sectional shape of the channel 890 can be slightly smaller than a cross-sectional shape of the spine 214. In this way, when the electrode assembly 800 is clamped together around the spine 214, the electrode assembly 800 forms a friction fit with the spine 214 to help prevent the electrode assembly 800 from sliding proximally and distally along a length of the spine 214.

FIG. 9 is a schematic pictorial illustration showing an exploded view of the electrode assembly 800, in accordance with another embodiment of the present invention. As illustrated, the electrode 840 can include a base portion 842 that can extend outwardly from the electrode 840. The first insulated housing portion 870 can further include a rim 872 that can extend inwardly near the top portion of the first insulated housing portion 870. To help secure the electrode 840 in the electrode assembly 800, the base portion 842 can be sized to have an outer perimeter length that is larger than the inner perimeter length of the rim 872. Thus, when the electrode 840 placed between the first and second insulated housing portions 870, 880 and the first insulated housing portion 870 is secured to the second insulated housing portion 880, the electrode 840 can be secured in place because the base portion 842 and the rim 872 prevent the electrode 840 from falling out of the first insulated housing portion 870.

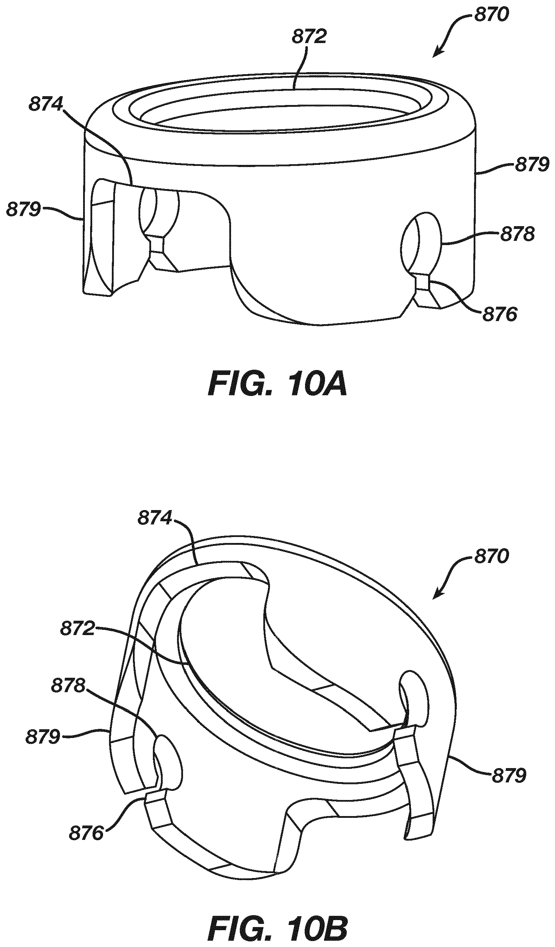

FIGs. 10A and 10B are schematic pictorial illustrations showing perspective views of the first insulated housing portion 870 of the electrode assembly 800. As illustrated, the first insulated housing portion 870 can include a channel opening 874 that can generally be a cut away portion of the first insulated housing portion 870 positioned between two sidewalls 879. The channel opening 874 can be sized to receive at least a portion of the spine 214. The channel opening 874 can include edges that are rounded to help reduce stress concentrations and the help reduce the likelihood that the first insulated housing portion 870 causes damage to the spine 214.

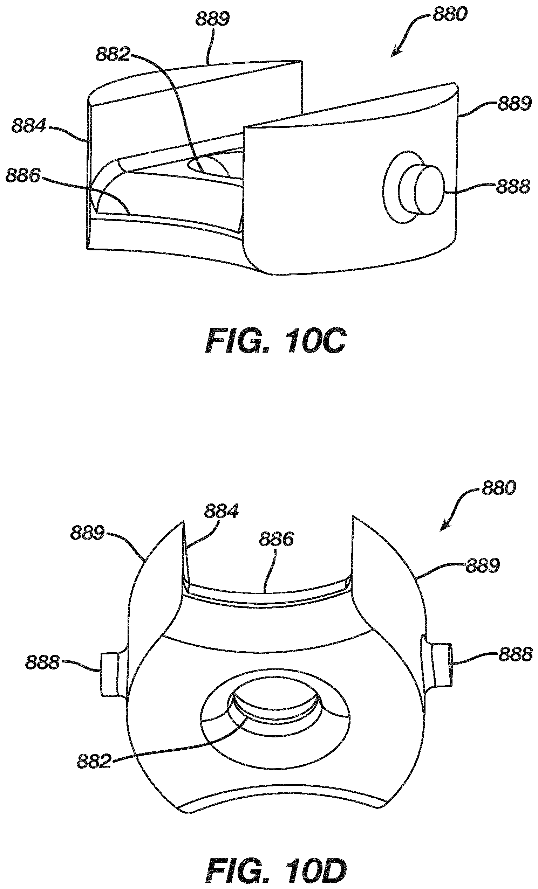

The first insulated housing portion 870 can further include a female connector 878 that is sized to receive a corresponding male connector 888 of the second insulated housing portion 880 (as shown and described in relation to FIGs. 10C and 10D). The female connector 878 can be positioned in or on a sidewall 879 of the first insulated housing portion 870 such that the female connector 878 can be positioned near a side of the spine 214 when the electrode assembly 800 is secured to the spine 214. The female connector 878 can include a narrow portion 876 that can be configured to allow the male connector 888 to be pushed into the female connector 878 but prevent the male connector 888 from being removed from the female connector 878 once assembled. For example, the narrow portion 876 can be sloped to a point that comprises a distance between opposing edges of the narrow portion 876 that is smaller than a diameter of the male connector 888. Thus, the narrow portion 876 can allow the male connector 888 to be slid through the narrow portion 876 in a first direction but prevent the male connector 888 from sliding through the narrow portion 876 in a second direction. The first insulated housing portion 870, the second insulated housing portion 880, or both, can be made from a resilient material to allow the narrow portion 876, the male connector 888, or both to deflect when the male connector 888 is pushed into the female connector 878.

FIGs. 10C and 10D are schematic pictorial illustrations showing perspective views of the second insulated housing portion 880 of the electrode assembly 800. As described previously, the second insulated housing portion 880 can include a male connector 888 that can protrude from the second insulated housing portion 880 and be configured to interlock with the female connector 878 of the first insulated housing portion 870. The second insulated housing portion 880 can further include channel portion 884 that can be sized to receive the spine 214 similar to the channel portion 874 of the first insulated housing portion 870. The channel portion 884 can be configured to align with the channel portion 874 such that the spine 214 can be positioned between the first insulated housing portion 870 and the second insulated housing portion 880 when assembled.

The channel portion 884 can include a rounded lip 886 that can be positioned proximate an outer edge of the channel portion 884 to help reduce stress concentrations and reduce the likelihood that the second insulated housing portion 880 damages the spine 214. The rounded lip 886 can include a rounded edge and be a generally concave shape such that the rounded lip 886 can permit the spine 214 to bend when transitioned between an expanded form and a collapsed form.

The second insulated housing portion 880 can include a wire aperture 882 that can be configured to receive a wire of the electrode assembly 800. For example, the wire can be disposed along the spine 214 and then pass through the wire aperture 882 and be electrically connected to the electrode 840. The wire can be welding, soldered, crimped, or otherwise connected to the electrode 840. The wire aperture 882 can include rounded edges to help reduce stress concentrations and reduce the likelihood that the second insulated housing portion 880 damages the wire. Alternatively, or in addition, the wire aperture 882 can be used for electrode 840 alignment. For example, the electrode 840 may have a pin-like emboss that would insert through an aperture included in the first insulated housing portion 870 and second insulated housing portion 880.

Although described as the first insulated housing portion 870 having the female connector 878 and the second insulated housing portion 880 having the male connector 888, one of skill in the art will appreciate that the two can be switched. In other words, the first insulated housing portion 870 can have the male connector 888 while the second insulated housing portion 880 can have a female connector 878 without departing from the scope of this disclosure. Furthermore, the electrode assembly 800 can have any number of female connectors 878 and male connectors 888 as would be suitable for the particular application.

Although the electrode assembly 800 and its components are illustrated in FIGs. 8A-10D as having a particular shape, one of skill in the art will appreciate that electrode assemblies 300 having other shapes are contemplated. For example, the electrode assembly 300 can comprise a generally circular, rectangular, triangular, polygonal, oval, or any other suitable shape without departing from the scope of this disclosure.

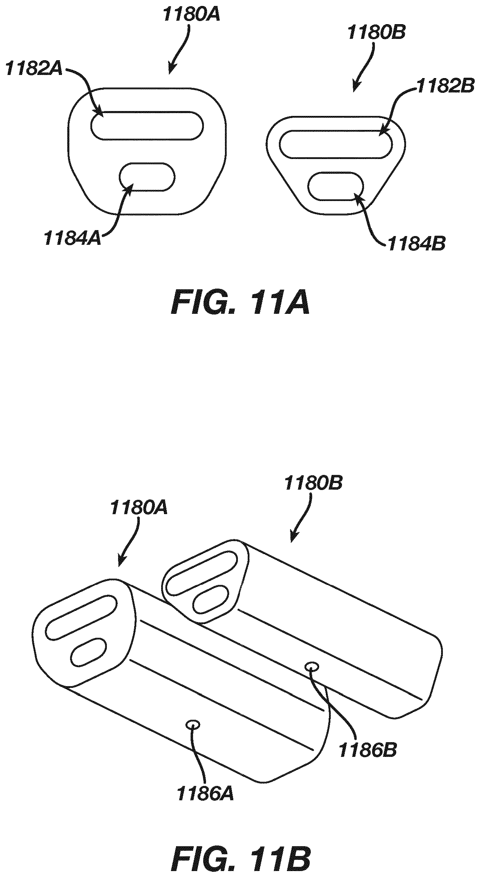

FIGs. 11A and 11B are schematic pictorial illustrations showing various insulative jackets 1180A, 1180B of a given medical device 22, in accordance with embodiments of the present invention. FIG. 11A is a front view while FIG. 11B is a perspective view of the insulative jackets 1180A, 1180B. The insulative jackets 1180A, 1180B can be made from a biocompatible, electrically insulative material such as polyamide-polyether (Pebax) copolymers, polyethelyene terephthalate (PET), urethanes, polyimide, parylene, silicone, etc. The insulative jackets 1180A, 1180B can further include a filler material such as PTFE, boron nitride, etc. The insulative jacket 1180A, 1180B can help to insulate a strut and/or wires passing through the insulative jacket 1180A, 1180B from the electrode 40 to prevent arcing from the electrode 40, 340, and/or 840 to the strut and/or wires passing through the insulative jacket 1180A, 1180B.

As illustrated in FIGs. 11A and 11B, the insulative jacket 1180A, 1180B, can include a cross-sectional shape that is substantially trapezoidal. The insulative jacket may consist of a single lumen or multi-lumen configuration. Multi-lumen jackets may be configured such that the alloy frame and wires share a single lumen while the second lumen may be used for irrigation. The alloy frame and wires may occupy separate lumens, also, as described. For these designs, the insulative jackets may be continuous (individual sleeves extending from proximal to distal end of each alloy frame strut), segmented (bridging between electrode gaps), or a combination of both. Furthermore, the insulative jacket 1180A, 1180B can include a first lumen 1182A, 1182B and a second lumen 1184A, 1184B. The first lumen 1182A, 1182B can be configured to receive a strut while the second lumen 1184A, 1184B can be configured to receive a wire, or vice-versa. In other examples, the first lumen 1182A, 1182B and the second lumen 1184A, 1184B can each be configured to receive one or more wires that can be connected to one or more electrodes 40. Furthermore, as illustrated in FIG. 10B, the insulative jacket 1180A, 1180B can include an aperture 1186A, 1186B through which a wire can be electrically connected to the electrode 40. Although illustrated in FIG. 10B as being proximate a bottom of the insulative jacket 1180A, 1180B, the aperture 1186A, 1186B can be positioned proximate a top or side of the insulative jacket 1180A, 1180B. Furthermore, the insulative jacket 1180A, 1180B can include multiple apertures 1186A, 1186B with each aperture being disposed on the same side of the insulative jacket (i.e., top, bottom, left, right) or on different sides of the insulative jacket depending on the application.

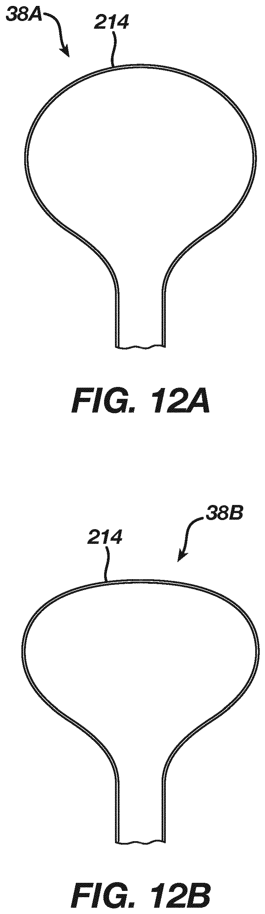

FIGs. 12A and 12B are schematic pictorial illustrations showing a side view of a spine 214 of a given medical device 22, in accordance with embodiments of the present invention. As will be appreciated, the spine 214 illustrated in FIGS. 12A and 12B is a single spine 214 and can be representative of the plurality of spines 214 of the basket assembly 38 described herein. In other words, the plurality of spines 214 forming the basket assembly 38 can each be configured to form the same or similar shape when in the expanded form such that the plurality of spines 214 together form a desired shape. To illustrate, the spine 214 as shown in FIG. 12A can be configured to form an approximately circular shape when in the expanded form. Thus, when combined with other spines 214 to form the basket assembly 38, the plurality of spines 214 can be configured to form an approximately spherical shape when the basket assembly 38 is in the expanded form. As another example, the spine 214 shown in FIG. 12B can be configured to form an approximately elliptical shape when in the expanded form. Thus, when combined with other spines 214 to form the basket assembly 38, the plurality of spines 214 can be configured to form an approximately oblate-spheroid shape when the basket assembly 38 is in the expanded form. Although not every variation of shape is shown or described herein, one skilled in the art will appreciate that the spines 214 can be further configured to form other various shapes as would be suitable for the particular application. Further, the medical device 22 can include one or more spines of alternative shapes which can form a shape having a profile with a periphery shaped similar to the spine 214 illustrated in FIG. 12A or 12B.

By including spines 214 configured to form various shapes when in the expanded form, the basket assembly 38 can be configured to position the various electrodes 40 attached to the spines 214 at various locations, with each location being nearer or farther from the distal end of the flexible insertion tube 30. For example, an electrode 40 attached to the spine 214 illustrated in FIG. 12A near the middle of the spine 214 would be farther from the distal end of the flexible insertion tube 30 than the spine 214 illustrated in FIG. 12B when the basket assembly 38 is in the expanded form.

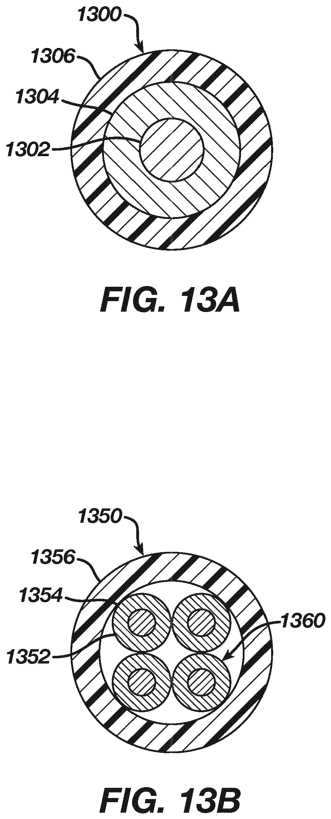

FIGs. 13A and 13B are schematic pictorial illustrations showing cross-sectional views of a given wire 1300, 1350 that can be connected to a given electrode 40, 340, 840, in accordance with an embodiment of the present invention. FIG. 13A illustrates a solid core wire 1300. FIG. 13B illustrates a stranded wire 1350. Each wire 1300, 1350 can extend through at least a portion of the tubular shaft 84. The solid core wire 1300 can include an electrically conductive core material 1302 and an electrically conductive cover material 1304 circumscribing electrically conductive core material 1302. Likewise, the stranded wire 1350 can include strands each including an electrically conductive core material 1352 and an electrically conductive cover material 1354 circumscribing the electrically conductive core material 1352. Each wire 1300, 1350 can include an insulative jacket 1306 circumscribing the conductors. The wires 1300, 1350 can be configured to withstand a voltage difference of adjacent wires sufficient to deliver IRE pulses. Preferably, the wires 1300, 1350 can withstand at least 900V, and more preferably at least 1,800V between adjacent wires. To reduce likelihood of dielectric breakdown between conductors of adjacent wires, the electrically conductive cover material 1304, 1354 can have a lower electrical conductivity compared to the core material 1302, 1352.

The insulative jacket 1306 can be configured to have a temperature rating between 150 and 200 degrees Centigrade so that the electrically insulative jacket 1306 melts or degrades (e.g., chars and crumbles) during soldering of the wire 1300 to the electrodes 40 (e.g., at a temperature of 300 degrees Centigrade) and therefore the insulative jacket 1306 of the wire 1300 does not need to be mechanically stripped. In other examples, the insulative jacket 1306 can have a temperature rating greater than 200 degrees Centigrade to prevent the electrically insulating material 1302 melting or degrading (e.g., charring and crumbling) during manufacture of the medical probe 22 and/or during use. The insulative jacket 1306 can be mechanically stripped from the wire 1300 prior to the wires 1300 being electrically connected to the electrodes 40.

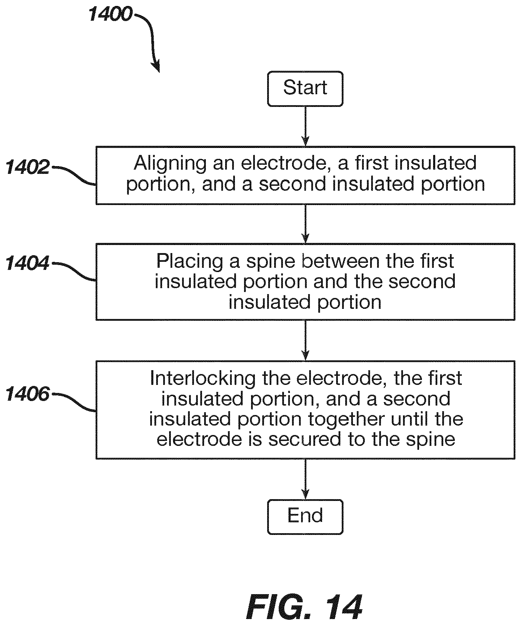

FIG. 14 is a flowchart illustrating a method 1400 of manufacturing a basket assembly 38, in accordance with an embodiment of the present invention. The method 1400 can include aligning 1402 a spine 214 of an expandable basket assembly 38 with a first insulated portion, and a second insulated portion. For example, as described herein, the method 1400 can include aligning 1402 a spine 214 with an electrode 340, a first washer 350, and a second wash 360. As another example, as described herein, the method 1400 can include aligning 1402 a spine 214 with an electrode 840, a first insulated housing portion 870, and a second insulated housing portion 880.

The method 1400 can further include placing 1404 the spine 214 between the first insulated portion and the second insulated portion. For example, as described herein, the spine 214 can be positioned between the first washer 350 and the second washer 360. As another example, as described herein, the spine 214 can be placed between the first insulated housing portion 870 and the second insulated housing portion 880 with the electrode 840 being disposed between the spine 214 and the first insulated housing portion 870.

The method 1400 can further include interlocking 1406 the electrode 340, 840, the first insulated portion (i.e., the first washer 350 or the first insulated housing portion 870), and the second insulated portion (i.e., the second washer 360 or the second insulated housing portion 880) together until the electrode is secured to the spine 214. For example, the method 1400 can include interlocking the electrode 340, the first washer 350, and the second washer 360 together until the electrode assembly 300 is secured in place by the swage fitting 344 engaging with the second washer 360. As another example, the method 1400 can include pushing the first insulated housing portion 870 together with the second insulated housing portion 880 until the male connectors 888 engages with the female connector 878 and the electrode assembly 800 is secured to the spine 214.

As will be appreciated by one skilled in the art, the method 1400 can include any of the various features of the disclosed technology described herein and can be varied depending on the particular configuration. Thus, the method 1400 should not be construed as limited to the particular steps and order of steps explicitly described herein.

The embodiments described above are cited by way of example, and the present invention is not limited by what has been particularly shown and described hereinabove. Rather, the scope of the invention is defined by the appended claims.

## Claims

1. A medical probe (22), comprising:
a tubular shaft (84) having a proximal end and a distal end, the tubular shaft extending along a longitudinal axis (L-L); and
an expandable basket assembly (38) coupled to the distal end of the tubular shaft, the basket assembly comprising:
at least one spine (214) extending along the longitudinal axis and configured to bow radially outward from the longitudinal axis when the expandable basket assembly is transitioned from a collapsed form to an expanded form; and
a plurality of electrode assemblies (300), each electrode assembly of the plurality of electrode assemblies being attached to the at least one spine and comprising:
an electrode (340);
a first electrically insulating portion (350) electrically isolating the electrode from the at least one spine; and
a second electrically insulating portion (360) electrically isolating the electrode from the at least one spine, the electrode, the first electrically insulating portion, and the second electrically insulating portion being interlocked onto the at least one spine such that the plurality of electrode assemblies are prevented from sliding proximally or distally along a length of the at least one spine,
wherein the at least one spine comprises a retainer ring (330) onto which a respective electrode assembly of the plurality of electrode assemblies is interlocked,
wherein the electrode comprises a protrusion (342) configured to interlock with the second electrically insulating portion, wherein the protrusion further comprises a swage fitting (344), wherein the protrusion is extending through the retainer ring, thereby preventing the electrode assembly from sliding along the length of the at least one spine.

2. The medical probe according to claim 1, wherein:
the first electrically insulating portion comprises a first washer (350); and
the second electrically insulating portion comprises a second washer (360).

3. The medical probe according to claim 2, wherein the first washer and the second washer are configured to prevent the electrode from contacting the at least one spine and to electrically isolate the electrode from the at least one spine.

4. The medical probe according to claim 3, wherein the second washer comprises a lip (364) configured to extend inwardly into a retainer ring of the at least one spine to prevent the electrode from contacting the at least one spine.

5. A medical probe (22), comprising:
A tubular shaft (84) having a proximal end and a distal end, the tubular shaft extending along a longitudinal axis (L-L); and
an expandable basket assembly (38) coupled to the distal end of the tubular shaft, the basket assembly comprising:
at least one spine (214) extending along the longitudinal axis and configured to bow radially outward from the longitudinal axis when the expandable basket assembly is transitioned from a collapsed form to an expanded form; and
a plurality of electrode assemblies (800), each electrode assembly of the plurality of electrode assemblies being attached to the at least one spine and comprising:
an electrode (840);
a first electrically insulating portion electrically isolating the electrode from the at least one spine, wherein the first electrically insulating portion comprises a first housing portion (870) comprising a female connector (878); and
a second electrically insulating portion electrically isolating the electrode from the at least one spine, wherein the second electrically insulating portion comprises a second housing portion (880) comprising a male connector (888), the electrode, the first electrically insulating portion, and the second electrically insulating portion being interlocked onto the at least one spine such that the plurality of electrode assemblies are prevented from sliding proximally or distally along a length of the at least one spine, wherein the first housing portion and the second housing portion are configured to interlock to secure the electrode to the at least one spine,
the female connector (878) configured receive the male connector and facilitate the first housing portion to interlock with the second housing portion.

6. The medical probe according to claim 5, the first housing portion comprising a rim (872) configured to receive the electrode.

7. The medical probe according to claim 6, the electrode further comprising a base portion (842) configured to retain the electrode in the rim of the first housing portion.

8. The medical probe according to claim 7, wherein the base portion comprises an outer perimeter length that is greater than an inner perimeter length of the rim.

9. The medical probe according to any of claims 5 to 8, wherein the electrode assembly includes a channel (890) sized to at least partially receive the spine such that the electrode assembly is secured to the spine by clamping around the spine.

10. A method of constructing a medical probe according to any preceding claim, the method comprising:
aligning the electrode, the first insulated portion, and the second insulated portion;
placing the spine of the basket catheter between the first insulated portion and the second insulated portion; and
interlocking the electrode, the first insulated portion, and the second insulated portion together to secure the electrode to the spine such that the first insulated portion and the second insulated portion electrically isolate the electrode from the spine and such that the electrode, the first insulated portion, and the second insulated portion are prevented from sliding proximally or distally along a length of the spine.

11. The method according to claim 10 when dependent upon any of claims 1 to 4, wherein interlocking the electrode, the first insulated portion, and the second insulated portion together comprises interlocking a portion of at least one of the electrode, the first insulated portion, and the second insulated portion through an aperture of the retainer ring of the spine.

12. The method according to claim 11, further comprising aligning the electrode, the first insulated portion, and the second insulated portion with the retainer ring.

13. The method according to claim 12, wherein interlocking the electrode, the first insulated portion, and the second insulated portion together comprises interlocking the protrusion of the electrode with the second insulated portion.

14. The method according to claim 10 when dependent upon any of claims 5 to 9, wherein interlocking the electrode, the first insulated portion, and the second insulated portion together comprises interlocking the first insulated housing portion and the second insulated housing portion to thereby secure the electrode to the spine.

15. The method according to claim 14 when dependent upon claim 7 or claim 8, wherein interlocking the electrode, the first insulated portion, and the second insulated portion together comprises retaining the base portion of the electrode in the rim.

## Patentansprüche

1. Medizinische Sonde (22), umfassend:
einen rohrförmigen Schaft (84), der ein proximales Ende und ein distales Ende aufweist, wobei sich der rohrförmige Schaft entlang einer Längsachse (L-L) erstreckt; und
eine expandierbare Korbanordnung (38), die mit dem distalen Ende der rohrförmigen Welle gekoppelt ist, die Korbanordnung umfassend:
mindestens eine Strebe (214), die sich entlang der Längsachse erstreckt und konfiguriert ist, um sich radial nach außen von der Längsachse zu wölben, wenn die expandierbare Korbbaugruppe von einer zusammengefalteten Form in eine expandierte Form überführt wird; und
eine Vielzahl von Elektrodenanordnungen (300), wobei jede Elektrodenanordnung der Vielzahl von Elektrodenanordnungen an der mindestens eine Strebe befestigt ist und umfasst:
eine Elektrode (340);
einen ersten elektrisch isolierenden Abschnitt (350), der die Elektrode von der mindestens einen Strebe elektrisch isoliert; und
einen zweiten elektrisch isolierenden Abschnitt (360), der die Elektrode von der mindestens einen Strebe elektrisch isoliert, wobei die Elektrode, der erste elektrisch isolierende Abschnitt und der zweite elektrisch isolierende Abschnitt auf der mindestens einen Strebe derart ineinandergreifend angeordnet sind, dass die Vielzahl von Elektrodenanordnungen daran gehindert wird, proximal oder distal entlang einer Länge der mindestens einen Strebe zu gleiten,
wobei die mindestens eine Strebe einen Haltering (330) umfasst, auf welchem eine jeweilige Elektrodenanordnung der Vielzahl von Elektrodenanordnungen ineinandergreifend angeordnet ist,
wobei die Elektrode einen Vorsprung (342) umfasst, der konfiguriert ist, um mit dem zweiten elektrisch isolierenden Abschnitt ineinander zu greifen, wobei der Vorsprung ferner eine Bördelverbindung (344) umfasst, wobei sich der Vorsprung durch den Haltering erstreckt, wodurch verhindert wird, dass die Elektrodenanordnung entlang der Länge der mindestens einen Strebe gleitet.

2. Medizinische Sonde nach Anspruch 1, wobei:
der erste elektrisch isolierende Abschnitt eine erste Unterlegscheibe (350) umfasst; und
der zweite elektrisch isolierende Abschnitt eine zweite Unterlegscheibe (360) umfasst.

3. Medizinische Sonde nach Anspruch 2, wobei die erste Unterlegscheibe und die zweite Unterlegscheibe konfiguriert sind, um zu verhindern, dass die Elektrode die mindestens eine Strebe kontaktiert, und die Elektrode von der mindestens einen Strebe elektrisch isolieren.

4. Medizinische Sonde nach Anspruch 3, wobei die zweite Unterlegscheibe eine Lippe (364) umfasst, die konfiguriert ist, um sich nach innen in einen Haltering der mindestens einen Strebe zu erstrecken, um zu verhindern, dass die Elektrode die mindestens eine Strebe kontaktiert.

5. Medizinische Sonde (22), umfassend:
einen rohrförmigen Schaft (84), der ein proximales Ende und ein distales Ende aufweist, wobei sich der rohrförmige Schaft entlang einer Längsachse (L-L) erstreckt; und
eine expandierbare Korbanordnung (38), die mit dem distalen Ende der rohrförmigen Welle gekoppelt ist, die Korbanordnung umfassend:
mindestens eine Strebe (214), die sich entlang der Längsachse erstreckt und konfiguriert ist, um sich radial nach außen von der Längsachse zu wölben, wenn die expandierbare Korbbaugruppe von einer zusammengefalteten Form in eine expandierte Form überführt wird; und
eine Vielzahl von Elektrodenanordnungen (800), wobei jede Elektrodenanordnung der Vielzahl von Elektrodenanordnungen an der mindestens einen Strebe befestigt ist und umfasst:
eine Elektrode (840);
einen ersten elektrisch isolierenden Abschnitt, der die Elektrode von der mindestens einen Strebe elektrisch isoliert, wobei der erste elektrisch isolierende Abschnitt einen ersten Gehäuseabschnitt (870) umfasst, der einen weiblichen Verbinder (878) umfasst; und
einen zweiten elektrisch isolierenden Abschnitt, der die Elektrode von der mindestens einen Strebe elektrisch isoliert, wobei der zweite elektrisch isolierende Abschnitt einen zweiten Gehäuseabschnitt (880) umfasst, der einen männlichen Verbinder (888) umfasst, wobei die Elektrode, der erste elektrisch isolierende Abschnitt und der zweite elektrisch isolierende Abschnitt auf der mindestens einen Strebe ineinandergreifend angeordnet sind, so dass die Vielzahl von Elektrodenanordnungen daran gehindert wird, proximal oder distal entlang einer Länge der mindestens einen Strebe zu gleiten, wobei der erste Gehäuseabschnitt und der zweite Gehäuseabschnitt konfiguriert sind, ineinanderzugreifen, um die Elektrode an der mindestens einen Strebe zu befestigen,
den weiblichen Verbinder (878), der konfiguriert ist, um den männlichen Verbinder aufzunehmen und das Ineinandergreifen des ersten Gehäuseabschnitts mit dem zweiten Gehäuseabschnitt zu erleichtern.

6. Medizinische Sonde nach Anspruch 5, wobei der erste Gehäuseabschnitt einen Rand (872) umfasst, der konfiguriert ist, um die Elektrode aufzunehmen.

7. Medizinische Sonde nach Anspruch 6, wobei die Elektrode ferner einen Basisabschnitt (842) umfasst, der konfiguriert ist, um die Elektrode in dem Rand des ersten Gehäuseabschnitts zu halten.

8. Medizinische Sonde nach Anspruch 7, wobei der Basisabschnitt eine äußere Umfangslänge umfasst, die größer ist als eine innere Umfangslänge des Rands.

9. Medizinische Sonde nach einem der Ansprüche 5 bis 8, wobei die Elektrodenanordnung einen Kanal (890) einschließt, der so dimensioniert ist, dass er die Strebe mindestens teilweise aufnimmt, so dass die Elektrodenanordnung an der Strebe durch Klemmen um die Strebe herum befestigt wird.

10. Verfahren zum Herstellen einer medizinischen Sonde nach einem der vorstehenden Ansprüche, wobei das Verfahren umfasst:
Ausrichten der Elektrode, des ersten isolierten Abschnitts und des zweiten isolierten Abschnitts;
Platzieren der Strebe des Korbkatheters zwischen dem ersten isolierten Abschnitt und dem zweiten isolierten Abschnitt; und
Ineinandergreifen der Elektrode, des ersten isolierten Abschnitts und des zweiten isolierten Abschnitts miteinander, um die Elektrode an der Strebe zu befestigen, derart, dass der erste isolierte Abschnitt und der zweite isolierte Abschnitt die Elektrode von der Strebe elektrisch isolieren und derart, dass die Elektrode, der erste isolierte Abschnitt und der zweite isolierte Abschnitt daran gehindert werden, proximal oder distal entlang einer Länge der Strebe zu gleiten.

11. Verfahren nach Anspruch 10, wenn abhängig von einem der Ansprüche 1 bis 4, wobei das Ineinandergreifen der Elektrode, des ersten isolierten Abschnitts und des zweiten isolierten Abschnitts miteinander das Ineinandergreifen eines Abschnitts von mindestens einem der Elektrode, des ersten isolierten Abschnitts und des zweiten isolierten Abschnitts durch eine Öffnung des Halterungsrings der Strebe umfasst.

12. Verfahren nach Anspruch 11, ferner umfassend das Ausrichten der Elektrode, des ersten isolierten Abschnitts und des zweiten isolierten Abschnitts mit dem Haltering.

13. Verfahren nach Anspruch 12, wobei das Ineinandergreifen der Elektrode, des ersten isolierten Abschnitts und des zweiten isolierten Abschnitts miteinander das Ineinandergreifen des Vorsprungs der Elektrode mit dem zweiten isolierten Abschnitt umfasst.

14. Verfahren nach Anspruch 10, wenn abhängig von einem der Ansprüche 5 bis 9, wobei das Ineinandergreifen der Elektrode, des ersten isolierten Abschnitts und des zweiten isolierten Abschnitts das Ineinandergreifen des ersten isolierten Gehäuseabschnitts und des zweiten isolierten Gehäuseabschnitts umfasst, um dadurch die Elektrode an der Strebe zu befestigen.

15. Verfahren nach Anspruch 14, wenn abhängig von Anspruch 7 oder Anspruch 8, wobei das Ineinandergreifen der Elektrode, des ersten isolierten Abschnitts und des zweiten isolierten Abschnitts das Halten des Basisabschnitts der Elektrode in dem Rand umfasst.

## Revendications

1. Sonde médicale (22), comprenant :
une tige tubulaire (84) ayant une extrémité proximale et une extrémité distale, la tige tubulaire s'étendant le long d'un axe longitudinal (L-L) ; et
un ensemble panier expansible (38) accouplé à l'extrémité distale de la tige tubulaire, l'ensemble panier comprenant :
au moins un élément d'armature (214) s'étendant le long de l'axe longitudinal et conçu pour s'incurver radialement vers l'extérieur à partir de l'axe longitudinal lorsque l'ensemble panier expansible effectue une transition d'une forme rétractée à une forme expansée ; et
une pluralité d'ensembles électrodes (300), chaque ensemble électrode de la pluralité d'ensembles électrodes étant fixé à l'au moins un élément d'armature et comprenant :
une électrode (340) ;
une première partie électriquement isolante (350) isolant électriquement l'électrode par rapport à l'au moins un élément d'armature ; et
une seconde partie électriquement isolante (360) isolant électriquement l'électrode par rapport à l'au moins un élément d'armature, l'électrode, la première partie électriquement isolante et la seconde partie électriquement isolante étant emboîtées sur l'au moins un élément d'armature de telle sorte que la pluralité d'ensembles électrodes sont empêchés de glisser proximalement ou distalement le long d'une longueur de l'au moins un élément d'armature,
dans laquelle l'au moins un élément d'armature comprend une bague de retenue (330) sur laquelle un ensemble électrode respectif de la pluralité d'ensembles électrodes est verrouillé,
dans laquelle l'électrode comprend une partie saillante (342) conçue pour s'emboîter avec la seconde partie électriquement isolante, dans laquelle la partie saillante comprend en outre un raccord de sertissage (344), dans laquelle la partie saillante s'étend à travers la bague de retenue, empêchant de ce fait l'ensemble électrode de glisser le long de la longueur de l'au moins un élément d'armature.

2. Sonde médicale selon la revendication 1, dans laquelle :
la première partie électriquement isolante comprend une première rondelle (350) ; et
la seconde partie électriquement isolante comprend une seconde rondelle (360).

3. Sonde médicale selon la revendication 2, dans laquelle la première rondelle et la seconde rondelle sont conçues pour empêcher l'électrode de venir en contact avec l'au moins un élément d'armature et pour isoler électriquement l'électrode par rapport à l'au moins un élément d'armature.

4. Sonde médicale selon la revendication 3, dans laquelle la seconde rondelle comprend une lèvre (364) conçue pour s'étendre vers l'intérieur dans une bague de retenue de l'au moins un élément d'armature pour empêcher l'électrode de venir en contact avec l'au moins un élément d'armature.

5. Sonde médicale (22), comprenant :
une tige tubulaire (84) ayant une extrémité proximale et une extrémité distale, la tige tubulaire s'étendant le long d'un axe longitudinal (L-L) ; et
un ensemble panier expansible (38) accouplé à l'extrémité distale de la tige tubulaire, l'ensemble panier comprenant :
au moins un élément d'armature (214) s'étendant le long de l'axe longitudinal et conçu pour s'incurver radialement vers l'extérieur à partir de l'axe longitudinal lorsque l'ensemble panier expansible effectue une transition d'une forme rétractée à une forme expansée ; et
une pluralité d'ensembles électrodes (800), chaque ensemble électrode de la pluralité d'ensembles électrodes étant fixé à l'au moins un élément d'armature et comprenant :
une électrode (840) ;
une première partie électriquement isolante qui isole électriquement l'électrode par rapport à l'au moins un élément d'armature, dans laquelle la première partie électriquement isolante comprend une première partie de logement (870) comprenant un connecteur femelle (878) ; et
une seconde partie électriquement isolante qui isole électriquement l'électrode par rapport à l'au moins un élément d'armature, dans laquelle la seconde partie électriquement isolante comprend une seconde partie de logement (880) comprenant un connecteur mâle (888), l'électrode, la première partie électriquement isolante et la seconde partie électriquement isolante étant emboîtées sur l'au moins un élément d'armature de telle sorte que la pluralité d'ensembles électrodes sont empêchés de glisser proximalement ou distalement le long d'une longueur de l'au moins un élément d'armature, dans laquelle la première partie de logement et la seconde partie de logement sont conçues pour s'emboîter pour fixer l'électrode à l'au moins un élément d'armature,
le connecteur femelle (878) conçu pour recevoir le connecteur mâle et faciliter l'emboîtement de la première partie de logement avec la seconde partie de logement.

6. Sonde médicale selon la revendication 5, la première partie de logement comprenant un rebord (872) conçu pour recevoir l'électrode.

7. Sonde médicale selon la revendication 6, l'électrode comprenant en outre une partie de base (842) conçue pour retenir l'électrode dans le rebord de la première partie de logement.

8. Sonde médicale selon la revendication 7, dans laquelle la partie de base comprend une longueur de périmètre externe qui est supérieure à une longueur de périmètre interne du rebord.

9. Sonde médicale selon l'une quelconque des revendications 5 à 8, dans laquelle l'ensemble électrode comporte un canal (890) dimensionné pour recevoir au moins partiellement l'élément d'armature de telle sorte que l'ensemble électrode est fixé à l'élément d'armature par serrage autour de l'élément d'armature.

10. Procédé de construction d'une sonde médicale selon l'une quelconque revendication précédente, le procédé comprenant :
l'alignement de l'électrode, de la première partie isolée et de la seconde partie isolée ;
la mise en place de l'élément d'armature du cathéter à panier entre la première partie isolée et la seconde partie isolée ; et
l'emboîtement de l'électrode, de la première partie isolée et de la seconde partie isolée ensemble pour fixer l'électrode à l'élément d'armature de telle sorte que la première partie isolée et la seconde partie isolée isolent électriquement l'électrode par rapport à l'élément d'armature et de telle sorte que l'électrode, la première partie isolée et la seconde partie isolée sont empêchées de glisser proximalement ou distalement le long d'une longueur de l'élément d'armature.

11. Procédé selon la revendication 10, prise en dépendance de l'une quelconque des revendications 1 à 4, dans lequel l'emboîtement de l'électrode, de la première partie isolée et de la seconde partie isolée ensemble comprend l'emboîtement d'une partie d'au moins l'une parmi l'électrode, la première partie isolée et la seconde partie isolée à travers une ouverture de la bague de retenue de l'élément d'armature.

12. Procédé selon la revendication 11, comprenant en outre l'alignement de l'électrode, de la première partie isolée et de la seconde partie isolée avec la bague de retenue.

13. Procédé selon la revendication 12, dans lequel l'emboîtement de l'électrode, de la première partie isolée et de la seconde partie isolée ensemble comprend l'emboîtement de la partie saillante de l'électrode avec la seconde partie isolée.

14. Procédé selon la revendication 10, prise en dépendance de l'une quelconque des revendications 5 à 9, dans lequel l'emboîtement de l'électrode, de la première partie isolée et de la seconde partie isolée ensemble comprend l'emboîtement de la première partie de logement isolée et de la seconde partie de logement isolée pour fixer de ce fait l'électrode à l'élément d'armature.

15. Procédé selon la revendication 14, prise en dépendance de la revendication 7 ou de la revendication 8, dans lequel l'emboîtement de l'électrode, de la première partie isolée et de la seconde partie isolée ensemble comprend la rétention de la partie de base de l'électrode dans le rebord.
